# FASCICULE DE BREVET EUROPEEN

(11) **EP 4 221 824 B1**
(45) Date de publication et mention de la délivrance du brevet: **13.08.2025**
(21) Numéro de dépôt: 21778164.0
(22) Date de dépôt: 30.09.2021
(51) Int. Cl.: A61N 1/37

(54) **DISPOSITIF DE SURVEILLANCE DE FONCTIONNEMENT D'UNE SONDE D'UN DISPOSITIF CARDIAQUE ACTIF IMPLANTABLE**
VORRICHTUNG ZUR ÜBERWACHUNG DES BETRIEBS EINER SONDE EINER IMPLANTIERBAREN AKTIVEN HERZVORRICHTUNG
DEVICE FOR MONITORING OPERATION OF A PROBE OF AN IMPLANTABLE ACTIVE CARDIAC DEVICE

(30) Priorité: 01.10.2020 FR 2010062
(43) Date de publication de la demande: 09.08.2023
(73) Titulaire: Sorin CRM SAS, 92140 Clamart (FR)
(72) Inventeur: GANDOIN, Anne-Sophie, 94260 Fresnes (FR); ROSSET, Nicolas, 92370 Chaville (FR); PLOUX, Sylvain, 33170 Gradignan (FR)
(74) Mandataire: Ungerer, Olaf
(86) Numéro de dépôt international: PCT/EP2021/077032
(87) Numéro de publication internationale: WO 2022/069680

(56) Documents cités:
- EP-A1- 1 857 143
- EP-A1- 3 081 257
- US-A1- 2014 350 620

## Description

### Domaine technique

La présente invention se rapporte à un dispositif de surveillance de fonctionnement d'une sonde d'un dispositif cardiaque actif implantable, en particulier d'un défibrillateur automatique implantable ou d'un défibrillateur pour resynchronisation cardiaque.

### Arrière-plan technique

Les sondes constituent la partie critique d'un système de dispositifs actifs implantables (DAI). En effet, les patients porteurs de DAI, en particulier d'un défibrillateur automatique implantable ou d'un défibrillateur pour resynchronisation cardiaque, sont exposés à un risque important de complications (allant jusqu'à 30%), dont la majorité sont liés à des chocs inappropriés. Ces chocs sont souvent dus à une altération de la sonde de défibrillation, fragilisant la détection du signal cardiaque et ainsi prenant en compte du bruit pour des arythmies.

Ces défaillances peuvent venir d'une abrasion suite aux frottements entre deux sondes (causant alors une perte d'isolant), d'une rupture des conducteurs ou encore d'un déplacement entraînant un mauvais contact entre l'extrémité de la sonde et la paroi cardiaque.

Le EP-A-1 857 143 divulgue un dispositif médical implantable actif de stimulation cardiaque, de resynchronisation, de cardioversion et/ou de défibrillation comportant des moyens de détection de fracture de sonde. Il s'agit de détecter la présence ou l'absence d'une contraction cardiaque, en partant du principe qu'à chaque cycle cardiaque réel correspond une seule contraction cardiaque. L'accélération endocardiaque est analysée, avantageusement en détectant la présence ou non d'un pic PEA I, pour confirmer la présence d'une activité mécanique du cœur sur détection d'une dépolarisation : une telle détection qui ne serait pas suivie par une activité mécanique du cœur peut avoir été générée par une perturbation produite par une fracture de sonde, elle est donc suspecte et doit être diagnostiquée comme telle.

Le EP-A-3 081 257 divulgue un dispositif médical implantable actif de stimulation cardiaque comprenant des moyens de détection d'un phénomène de remodelage ou remodelage inverse d'un patient. Le dispositif opère par analyse morphologique comparative de signaux de dépolarisation recueillis en rythme spontané sur des voies respectives distinctes, avec deux composantes temporelles combinées en une unique caractéristique 2D paramétrique de vectogramme VGM. Des moyens d'analyse historique évaluent la variation au cours du temps d'un paramètre descripteur du VGM courant par rapport à un VGM de référence antérieur mémorisé. Cette variation est comparée à des seuils prédéterminés pour diagnostiquer l'apparition d'un remodelage ou remodelage inverse chez le patient, et/ou détecter une rupture de sonde ou la survenue d'une ischémie.

Le US-A-2014/350620 divulgue un dispositif médical implantable capable de détecter des signaux cardiaques et de délivrer des thérapies de stimulation électrique cardiaque, et permettant de détecter un événement de court-circuit. Un signal est détecté par un module de détection couplé à des électrodes. Un contrôleur détecte un événement de court-circuit en réponse à une pente du signal détecté dépassant un seuil de court-circuit.

### Description de l'invention

La présente invention a pour objet d'améliorer la prédiction d'une sonde défaillante.

L'objet de la présente invention est atteint au moyen d'un dispositif de surveillance de fonctionnement d'une sonde d'un dispositif cardiaque actif implantable selon la revendication 1, en particulier un défibrillateur automatique implantable ou un défibrillateur pour resynchronisation cardiaque, comprenant un dispositif de détermination de paramètres pour déterminer des valeurs d'une pluralité de paramètres caractérisant la sonde. Le dispositif de surveillance comprend une unité de traitement configurée pour déterminer des valeurs représentatives d'au moins un paramètre de la pluralité de paramètres caractérisant la sonde en se basant sur au moins deux échelles de temps différentes. L'unité de traitement est en outre configurée pour comparer une valeur dite valeur d'analyse d'au moins un paramètre de la pluralité de paramètres caractérisant la sonde avec les valeurs représentatives dudit paramètre.

En comparant la valeur d'analyse aux valeurs représentatives selon deux échelles de temps différentes d'au moins un paramètre caractérisant la sonde, le présent dispositif est configuré pour détecter une défaillance de la sonde.

Les paramètres auxquels est comparée la valeur d'analyse étant caractéristiques de la sonde, un écart entre les valeurs représentatives et la valeur d'analyse indique un problème de sonde, et non une anomalie cardiaque par exemple. Ainsi, le présent dispositif permet d'améliorer la prédiction d'une sonde défaillante.

La comparaison en considérant deux échelles de temps différentes permet d'affiner davantage la fiabilité de la détection d'une défaillance de la sonde.

La présente invention, relative à un dispositif de surveillance de fonctionnement d'une sonde d'un dispositif cardiaque actif implantable, peut être davantage améliorée grâce aux modes de réalisation suivants.

Selon un mode de réalisation, une première valeur représentative peut être une moyenne d'un premier nombre prédéfini de valeurs représentatives déterminées avant la valeur d'analyse qui est comparée au moyen de l'unité de traitement.

Ladite première valeur représentative est donc déterminée de manière à représenter une tendance d'un paramètre caractérisant la sonde qui est antérieure à la valeur d'analyse. Ladite première valeur représentative peut ainsi constituée une valeur comparative.

Selon un mode de réalisation, une deuxième valeur représentative peut être une moyenne d'un deuxième nombre prédéfini de valeurs représentatives déterminées avant la valeur d'analyse qui est comparée au moyen de l'unité de traitement, ledit deuxième nombre prédéfini étant supérieur au premier nombre prédéfini.

Ainsi, deux valeurs représentatives peuvent être déterminées sur la base de deux échelles de temps différentes.

La comparaison de la valeur d'analyse en considérant deux échelles de temps différentes permet d'affiner davantage la fiabilité de la détection d'une défaillance de la sonde. En effet, la détermination d'une défaillance de la sonde peut dépendre de l'échelle de temps considérée par rapport à la valeur d'analyse.

Selon un mode de réalisation, une troisième valeur représentative peut être une moyenne glissante basée sur une moyenne d'un troisième nombre prédéterminé de valeurs représentatives déterminées avant la valeur d'analyse qui est comparée au moyen de l'unité de traitement, ladite moyenne d'un troisième nombre prédéterminé de valeurs correspondant à un paramètre de la pluralité des paramètres. La moyenne glissante est un type de moyenne statistique particulièrement adaptée pour analyser des séries temporelles, notamment en supprimant les fluctuations transitoires de façon à en souligner les tendances à plus long terme.

De plus, le dispositif est ainsi capable de déterminer trois valeurs représentatives sur la base de trois échelles de temps différentes.

Selon un mode de réalisation, l'unité de traitement peut être configurée lors de la détermination des valeurs représentatives de manière à ce qu'une valeur parmi les valeurs de la pluralité de paramètres caractérisant la sonde qui dépasse une valeur limite prédéfinie n'est pas prise en compte.

Ainsi, il est possible d'écarter des valeurs qui ne seraient pas considérées utilisables ou comme étant comprises dans une gamme de valeurs viables. De telles valeurs considérées comme anormales seraient alors avantageusement écartées de la détermination des valeurs représentatives afin d'améliorer leur fiabilité.

Selon un mode de réalisation, l'unité de traitement peut être configurée pour comparer la valeur d'analyse d'au moins un paramètre de la pluralité de paramètres caractérisant la sonde avec les valeurs représentatives dudit au moins un paramètre, la valeur la plus récente par rapport à la valeur d'analyse qui est prise en compte pour la détermination des valeurs représentatives étant comprise dans un premier intervalle de temps prédéterminé.

Il est ainsi possible de déterminer un premier intervalle de temps qui permet de ne prendre en considération que des valeurs pour la détermination des valeurs représentatives à partir d'un évènement qui n'est pas considéré comme trop ancien par rapport à la valeur d'analyse.

De ce fait, la fiabilité du dispositif, et donc de la prédiction d'une sonde défaillante, est davantage améliorée.

Selon un mode de réalisation, l'unité de traitement peut être configurée pour comparer la valeur d'analyse d'au moins un paramètre de la pluralité de paramètres caractérisant la sonde avec les valeurs représentatives dudit au moins un paramètre, la valeur la plus ancienne par rapport à la valeur d'analyse qui est prise en compte pour la détermination des valeurs représentatives étant comprise dans un deuxième intervalle de temps prédéterminé.

Il est ainsi possible de déterminer un deuxième intervalle de temps qui permet de ne prendre en considération que des valeurs pour la détermination des valeurs représentatives en remontant temporellement jusqu'à un événement qui n'est pas considéré comme trop anciens par rapport à la valeur d'analyse.

De ce fait, la fiabilité du dispositif, et donc de la prédiction d'une sonde défaillante, est davantage améliorée.

Selon un mode de réalisation, un paramètre peut être un paramètre parmi une amplitude du signal de détection, une continuité de la sonde, un pourcentage journalier de détection, un nombre de fibrillations ventriculaires non soutenues, un nombre de fibrillations ventriculaires non traitées, un nombre de fibrillations ventriculaires traitées, un nombre d'extrasystoles isolées, un nombre d'extrasystoles totales, une impédance de la sonde et un seuil de stimulation. Ainsi, le présent dispositif est configuré pour déterminer et ne prendre en compte que des paramètres caractérisant une sonde.

Selon un mode de réalisation, la pluralité de paramètres caractérisant la sonde peut comprendre au moins deux paramètres différents, en particulier au moins trois paramètres différents.

La prise en compte de deux paramètres différents, de préférence trois, caractérisant la sonde permet d'améliorer davantage la fiabilité et la sensibilité du présent dispositif de surveillance de sonde.

Selon un mode de réalisation, le dispositif peut en outre comprendre une unité d'alerte pour émettre une alerte quand la valeur d'analyse dépasse de manière croissante ou décroissante une valeur limite d'au moins une valeur représentative ou/et un seuil limite d'au moins un paramètre de la pluralité des paramètres.

Le dispositif est ainsi configuré pour émettre une alerte lorsqu'une défaillance de sonde est déterminée par le dépassement de la valeur d'analyse. Le terme « seuil limite » d'un paramètre regroupe deux aspects : à la fois celle de valeur limite (par exemple : le paramètre est au-delà d'une valeur limite) et celle de variation limite (par exemple : le paramètre varie de plus de cette variation limite).

Selon un mode de réalisation, chacun des paramètres de la pluralité de paramètres peut avoir respectivement un seuil limite, les seuils limites étant groupés dans un premier groupe de seuils limites pour lequel l'unité d'alerte est configurée pour émettre une alerte en cas de dépassement d'un seuil limite d'un seul paramètre, ou un second groupe de seuils limites pour lequel l'unité d'alerte est configurée pour émettre une alerte en cas de dépassement concomitant des seuils limites d'au moins deux différents paramètres.

Ainsi, le présent dispositif est capable de différencier la nécessité d'émettre une alerte ou pas en fonction des paramètres dont le seuil limite est franchi. Ainsi, ne sont émises que des alertes considérées comme justifiées. Tel indiqué ci-dessus, le terme « seuil limite » d'un paramètre regroupe deux aspects : à la fois celle de valeur limite (par exemple : le paramètre est au-delà d'une valeur limite) et celle de variation limite (par exemple : le paramètre varie de plus de cette variation limite).

Selon un mode de réalisation, un seuil limite d'un paramètre assigné au deuxième groupe peut être transféré dans le premier groupe si le dépassement dudit seuil limite a lieu successivement un nombre prédéterminé de fois.

Ainsi, il est possible d'adapter la sensibilité et la spécificité des alertes au cours de la surveillance de la sonde en fonction des dépassements de seuil limite identifiés.

Selon un mode de réalisation, les seuils limites relatifs à l'impédance de sonde, à la continuité de la sonde et au nombre d'extrasystoles totales peuvent faire partie du premier groupe, et les seuils limites relatifs à l'amplitude d'un signal de détection, au pourcentage de détection, au seuil de stimulation, au nombre d'extrasystoles isolées, au nombre de fibrillations ventriculaires traitées, au nombre de fibrillations ventriculaires soutenues mais non traitées, et au nombre de fibrillations ventriculaires non soutenues peuvent faire partie du deuxième groupe.

Ainsi, le présent dispositif est adapté pour discriminer les seuils limites relatifs à des paramètres qui se suffisent à eux-mêmes pour justifier l'émission d'une alerte.

Selon un mode de réalisation, une valeur de pondération peut être attribuée à chaque paramètre du second groupe et dans lequel l'unité d'alerte peut être configurée pour déclencher une alerte quand la somme des valeurs de pondération des au moins deux paramètres dépasse un nombre prédéterminé.

La pondération des paramètres les uns par rapport aux autres permet de déterminer si leur dépassement respectif de seuil limite en concomitance est suffisant pour déclencher l'émission d'une alerte.

Selon un mode de réalisation, l'unité d'alerte peut comprendre une unité de mémoire configurée pour sauvegarder un dépassement de seuil limite pendant une durée déterminée et pour le supprimer après l'écoulement de ladite durée déterminée.

La prise en considération d'évènements précédents qui ont ou ont été susceptibles de déclencher l'émission d'une alerte permet d'améliorer davantage la prédiction d'une sonde défaillante.

Selon un mode de réalisation, un paramètre de la pluralité de paramètres peut comprendre un premier seuil limite et un deuxième seuil limite, le premier seuil limite faisant partie du premier groupe et le deuxième seuil limite faisant partie du deuxième groupe.

Chacun des seuils limites peut correspondre par exemple à des seuils limites se rapportant à des échelles de temps différentes. Un premier seuil limite peut ainsi se rapporter à une valeur temporelle discrète alors que le deuxième seuil limite peut se rapporter à une variation du paramètre.

Selon un mode de réalisation, des seuils limites parmi les seuils limites du deuxième groupe peuvent être liés entre eux et d'autres peuvent ne pas être liés entre eux, de manière à ce que l'unité d'alerte peut être configurée pour déclencher une alerte en présence d'au moins deux dépassements de seuils limites parmi des seuils du deuxième groupe qui ne sont pas liés entre eux.

Des seuils limites de paramètres peuvent être liés entre eux dans le cas où ils reflètent un même problème (par exemple la proportion de détection et l'amplitude de détection). Le dépassement de deux seuils limites liés entre eux n'est ainsi pas considéré comme suffisant pour émettre une alerte.

Il peut ainsi être nécessaire d'avoir la présence d'au moins deux dépassements de seuils limites parmi des seuils du deuxième groupe qui ne sont pas liés entre eux (comme par exemple le nombre d'épisodes de fibrillation ventriculaire par jour et le seuil de stimulation) pour émettre une alerte.

### Description des figures

L'invention et ses avantages seront expliqués plus en détail dans ce qui suit au moyen de modes de réalisation préférés et en s'appuyant notamment sur les figures d'accompagnement suivantes, dans laquelle :
- La **Figure 1**: représente un dispositif de surveillance de fonctionnement selon la présente invention.
- La **Figure 2**: représente l'analyse sur la variation d'un paramètre sur une deuxième échelle de temps dite « à moyen terme ».
- La **Figure 3**: représente l'analyse sur la variation d'un paramètre sur une troisième échelle de temps dite « à long terme ».
- La **Figure 4a**: représente une première partie d'un organigramme relatif à l'analyse de variations de valeurs d'un paramètre sur trois échelles de temps différentes selon la présente invention et à la levée d'avertissement.
- La **Figure 4b**: représente une seconde portion de l'organigramme illustré à la Figure 4a.
- La **Figure 5**: représente un organigramme relatif à un déclenchement d'alerte en fonction des avertissements levés selon la présente invention.
- La **Figure 6**: représente un tableau de pondération de la suffisance des avertissements entre eux.

L'invention va maintenant être décrite plus en détail en utilisant des modes de réalisation avantageux d'une manière exemplaire et en référence aux figures. Les modes de réalisation décrits sont simplement des configurations possibles et il faut garder à l'esprit que les caractéristiques individuelles telles que décrites ci-dessus peuvent être fournies indépendamment les unes des autres ou peuvent être omises tout à fait lors de la mise en œuvre de la présente invention.

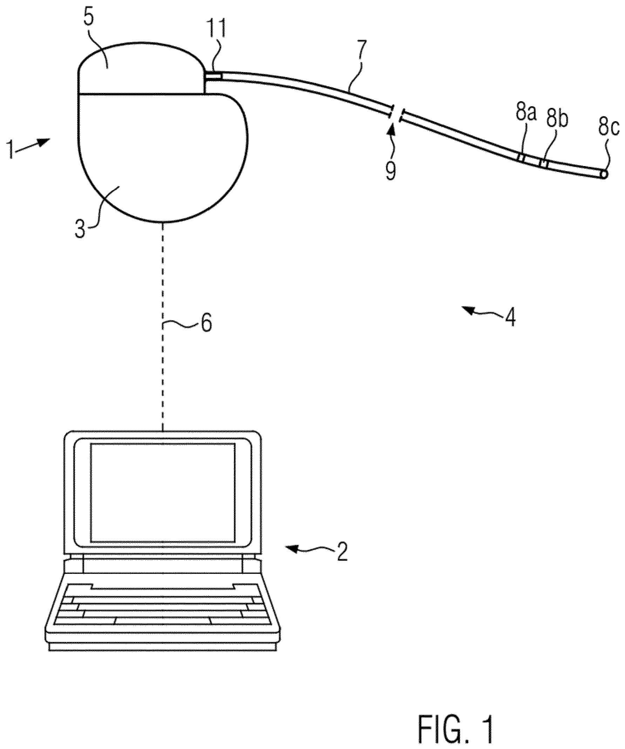

La Figure 1 illustre un dispositif cardiaque actif implantable 1 et une unité de traitement 2 formant un dispositif de surveillance de fonctionnement d'une sonde 4 selon la présente invention.

Le dispositif cardiaque actif implantable 1 peut être un défibrillateur automatique implantable ou un défibrillateur adapté pour la resynchronisation cardiaque.

Le dispositif cardiaque implantable 1 comprend un boîtier 3. Le boîtier 3 comprend notamment des circuits électroniques et une pile, par exemple de type lithium/iode. Le boîtier 3 comprend également une partie connecteur 5 dans laquelle peut être insérée puis vissée une sonde implantable 7.

Bien que la Figure 1 représente un exemple de dispositif cardiaque actif implantable comprenant une sonde implantable 7, il faut garder à l'esprit que dans une variante (non-représentée) plusieurs sondes implantables peuvent être connectées à la partie connecteur 5 du boîtier 3. Le dispositif de surveillance 4 de fonctionnement d'une sonde est configuré pour un dispositif cardiaque actif implantable comprenant plusieurs sondes. Le dispositif de surveillance 4 de fonctionnement d'une sonde est ainsi configuré pour déterminer une défaillance résultant d'une abrasion suite aux frottements entre deux sondes, causant une perte au moins partielle de leur isolant.

La sonde implantable 7 comprend plusieurs électrodes 8a, 8b, 8c - le nombre d'électrodes illustré dans la Figure 1 n'étant pas limitatif - qui constituent des moyens de détection et de stimulation et/ou défibrillation du dispositif cardiaque implantable 1.

La sonde implantable 7 peut être une sonde de défibrillation.

La sonde implantable 7 est configurée pour mesurer des valeurs d'une pluralité de paramètres la caractérisant, telles que des valeurs d'impédance par exemple.

Selon la présente invention, la pluralité de paramètres caractérisant la sonde implantable 7 peut au moins comprendre : l'amplitude du signal de détection, la continuité de la sonde, un pourcentage journalier de détection, un nombre de fibrillations ventriculaires non soutenues, un nombre de fibrillations ventriculaires non traitées, un nombre de fibrillations ventriculaires traitées, un nombre d'extrasystoles isolées, un nombre d'extrasystoles totales, une impédance de la sonde et un seuil de stimulation.

Une extrasystole isolée est définie comme un cycle cardiaque avec une seule extrasystole.

En outre, dans un mode de réalisation dans lequel la sonde implantable 7 est une sonde de défibrillation, les paramètres suivants peuvent également être considérés : la continuité de la sonde de défibrillation, le nombre de fibrillations ventriculaires traitées, le nombre de fibrillations ventriculaires soutenues mais non traitées et le nombre de fibrillations ventriculaires non soutenues.

Une fibrillation ventriculaire traitée est définie comme une fibrillation ventriculaire qui persiste en tant que telle et qui a été traitée par choc électrique.

Une fibrillation ventriculaire non traitée est définie comme une fibrillation ventriculaire qui persiste en tant que telle mais qui n'a pas été traitée par choc électrique.

Une fibrillation ventriculaire non soutenue est définie comme une fibrillation ventriculaire qui ne persiste pas et qui n'a pas été traitée par choc électrique.

Notons que bien que tous les paramètres susmentionnés caractérisant la sonde ne soient pas disponibles pour tous les types de sondes, et comme il sera expliqué davantage dans ce qui suit, cela n'influence pas l'analyse multifactorielle implémentée par le dispositif de surveillance 4 de fonctionnement d'une sonde de la présente invention.

Le dispositif cardiaque implantable 1 fournit ainsi un dispositif de détermination de paramètres pour déterminer des valeurs d'une pluralité de paramètres caractérisant la sonde implantable 7.

Les brisures de lignes 9 indiquent que la longueur de la sonde implantable 7 n'est pas entièrement représentée à la Figure 1 pas soucis d'échelle de dessin.

La sonde implantable 7 est connectée à la partie connecteur 5 du boîtier 3 au moyen d'une broche 11. Un vissage partiel ou une introduction insuffisante de la broche 11 dans la partie connecteur 5 du dispositif cardiaque implantable 1 peut causer des problèmes de connectique.

Tel qu'il sera expliqué plus en détail dans ce qui suit, le dispositif de surveillance 4 de fonctionnement d'une sonde selon la présente invention est configuré pour détecter ce type de défaillances.

Pour ce faire, le dispositif de surveillance 4 de fonctionnement d'une sonde selon la présente invention comprend en outre une unité de traitement 2.

L'unité de traitement 2 peut être implémenté dans le dispositif cardiaque implantable 1 ou dans un dispositif externe, comme un ordinateur.

Le dispositif cardiaque implantable 1 et l'unité de traitement 2 sont configurés pour communiquer entre eux, par exemple pas télémétrie 6.

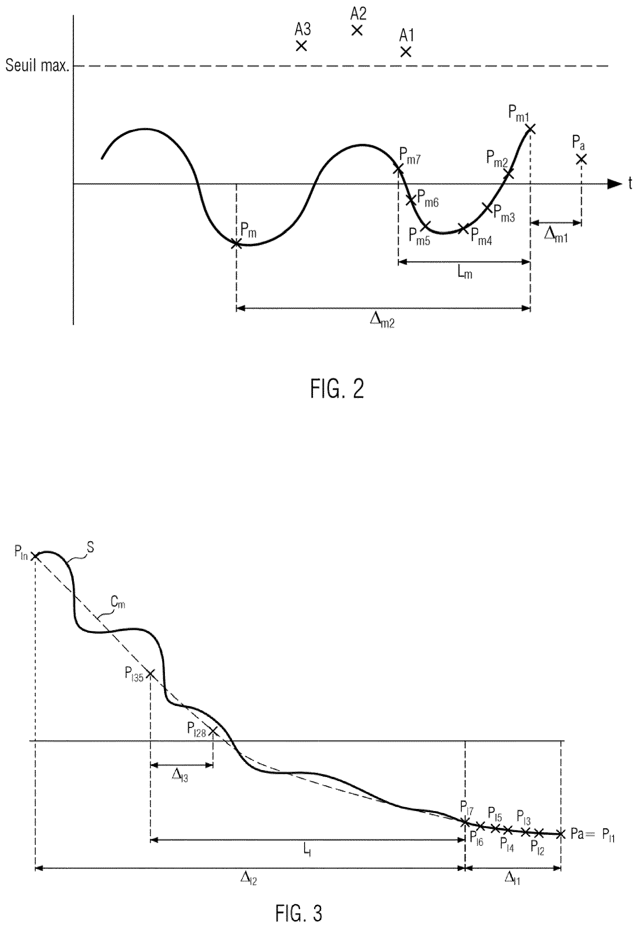

L'unité de traitement 2 est configurée pour déterminer des valeurs représentatives d'au moins un paramètre de la pluralité de paramètres caractérisant la sonde implantable 7 en se basant sur au moins deux échelles de temps différentes, en particulier trois échelles de temps. L'analyse de variations des valeurs d'un paramètre sur des échelles de temps différentes est davantage décrite en référence aux Figures 2 et 3.

L'unité de traitement 2 est en outre configurée pour comparer une valeur dite valeur d'analyse d'au moins un paramètre de la pluralité de paramètres caractérisant la sonde implantable 7 avec les valeurs représentatives dudit paramètre.

Selon la présente invention, l'analyse de chacun des paramètres caractérisant la sonde implantable 7 peut être effectuée selon plusieurs facteurs comme un seuil maximal ou minimal, une variation absolue ascendante ou descendante dite « à court terme » (par exemple sur un jour), une variation absolue ou relative ascendante ou descendante dite « à moyen terme » (par exemple sur une semaine) et une variation relative ascendante ou descendante dite « à long terme » (par exemple sur un mois). L'étude des variations des paramètres est décrite en référence aux Figures 2, 3 et 4.

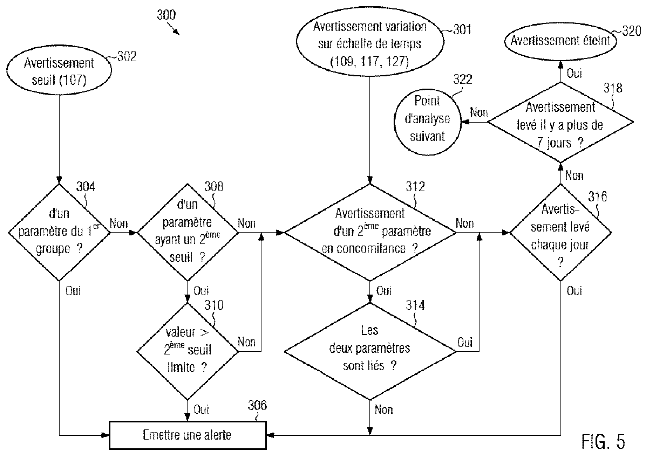

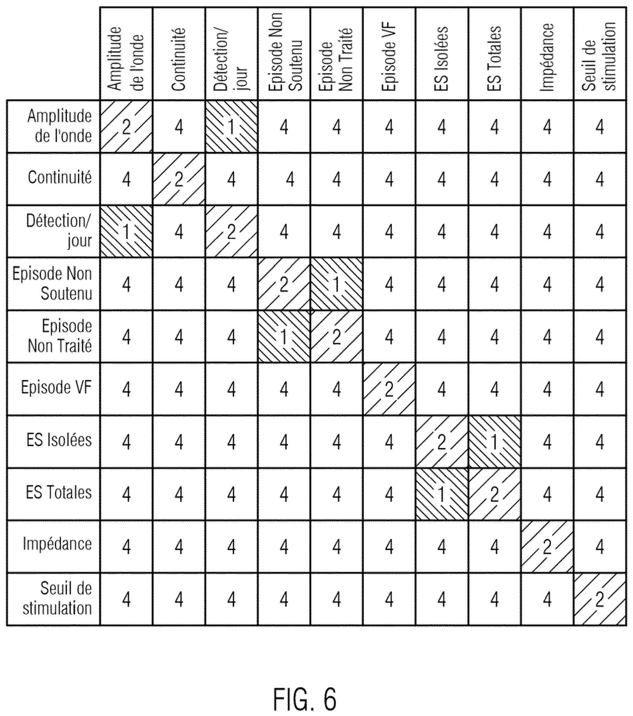

Secondement, une combinaison de toutes ces analyses relatives aux paramètres caractérisant la sonde implantable est effectuée afin de lever une alerte, ce qui est décrit en référence aux Figures 5 et 6.

Notons que l'unité de traitement 2 est configurée lors de la détermination des valeurs représentatives de manière à ce qu'une valeur parmi les valeurs de la pluralité de paramètres caractérisant la sonde qui dépasse une valeur limite prédéfinie n'est pas prise en compte. Une valeur dépassant une telle valeur limite prédéfinie est qualifiée de « point non utilisable » c'est-à-dire à un point dont la valeur est en dehors d'une gamme de valeurs viables ou dont la valeur n'est pas disponible. Au contraire, la valeur dépassant une telle valeur limite prédéfinie est qualifiée de « point anormal » lorsqu'il s'agit d'un point dont la valeur dépasse un seuil maximal ou minimal. Les autres valeurs, qui ne dépassent pas de valeur limite prédéfinie, sont considérées comme « normales » et donc utilisables pour l'analyse sur la variation.

L'analyse sur la variation sur une première échelle de temps dite « à court terme » se fait en analysant les variations entre un point maximal et un point minimal d'une même journée. Par exemple, l'unité de traitement 2 prend en compte quatre mesures d'impédance au cours d'une journée. Dans une variante, l'unité de traitement 2 prend également en considération d'autres paramètres comme le seuil de stimulation ou encore l'amplitude de détection. L'unité de mesure peut prendre plus ou moins de quatre mesures d'un paramètre sur une journée.

L'unité de traitement 2 comprend en outre une unité d'alerte (non représentée sur la Figure 1). L'unité d'alerte est configurée pour émettre une alerte quand la valeur d'analyse dépasse de manière croissante ou décroissante une valeur limite d'au moins une valeur représentative ou/et un seuil limite d'au moins un paramètre de la pluralité des paramètres. Le terme « seuil limite » d'un paramètre regroupe deux aspects : à la fois celle de valeur limite (par exemple : le paramètre est au-delà d'une valeur limite) et celle de variation limite (par exemple : le paramètre varie de plus de cette variation limite).

L'unité de traitement 2 comprend également une unité de mémoire (non représentée sur la Figure 1) au moyen de laquelle des données peuvent être sauvegardées.

La Figure 2 représente l'analyse sur la variation selon une deuxième échelle de temps dite « à moyen terme ».

La deuxième échelle de temps selon la présente invention est différente de la première échelle de temps en ce qu'elle se rapporte à l'analyse de variation sur plus d'une journée, en particulier sur une semaine.

Les analyses de variations (relative ou absolue) sur la deuxième échelle de temps dite « à moyen terme » s'effectuent entre un point d'analyse Pa et une ligne de base Lm dite ligne de base moyen terme.

Le point d'analyse Pa correspond à un point représentatif de la moyenne journalière.

La ligne de base Lm correspond à un nombre n de derniers points chacun représentatif de la moyenne journalière. Cette ligne de base Lm n'intègre que des points qualifiés de « normaux », c'est-à-dire que les points A1, A2, A3 de la Figure 2 sont exclus de cette ligne de base Lm car ils ont chacun une valeur dépassant un seuil maximal (représenté par une ligne horizontale en pointillé sur la Figure 2). Des points dépassant un seuil minimal peuvent également être exclus de la ligne de base Lm.

La ligne de base Lm étant une ligne de base Lm « moyen terme », c'est-à-dire relative à une semaine, la ligne de base Lm moyen terme correspond aux sept derniers points Pm1 à Pm7 qualifiés de normaux de la ligne de base Lm. Tel qu'expliqué ci-dessus, chacun des sept points Pm1 à Pm7 correspond à une moyenne journalière.

Sur l'exemple de la Figure 2, Pm1 représente le point le plus récent de la ligne de base Lm moyen terme par rapport au point d'analyse Pa. Pm7 représente quant à lui le point le plus ancien de la ligne de base Lm moyen terme par rapport au point d'analyse Pa.

Afin de ne pas comparer le point d'analyse Pa à des évènements jugés trop anciens, il peut être déterminé un premier intervalle de temps Δm1 entre le point d'analyse Pa et le point Pm1 le plus récent de la ligne de base Lm moyen terme. Ce premier intervalle de temps Δm1 peut correspondre à une durée de sept jours. Dans le cas inverse, l'analyse de variations peut être suspendue.

En outre, il peut être déterminé un deuxième intervalle de temps Δm2 entre le point Pm1 le plus récent de la ligne de base Lm moyen terme et un point Pm de la ligne de base Lm qui pourrait correspondre au point le plus ancien de la ligne de base Lm moyen terme. Ce deuxième intervalle de temps Δm2 peut correspondre à une durée de quatorze jours. Dans le cas inverse, l'analyse de variations peut être suspendue.

La Figure 3 représente l'analyse sur la variation de valeurs sur une troisième échelle de temps dite « à long terme ».

L'unité de traitement 2 du dispositif de surveillance 4 de fonctionnement d'une sonde selon la présente invention considère un signal S obtenu au moyen du dispositif de détermination de paramètres dudit dispositif de surveillance 4. Le signal S peut être un signal brut ou un signal traité par des moyens de filtrage connus.

L'unité de traitement 2 est configuré pour opérer une moyenne glissante, en particulier sur sept points, sur le signal S. La courbe Cm de la Figure 3 représente la courbe moyennée ainsi obtenue. Le moyennage permet d'éviter des altérations dues à des variations dite « à court terme », c'est-à-dire par les variations sur une journée.

La courbe Cm de la Figure 3 correspond à la courbe prise en considération pour l'analyse de la variation sur la troisième échelle de temps dite long terme.

Le moyennage créant un décalage, la courbe Cm peut être recentrée sur trois jours afin de recaler la courbe Cm.

La courbe Cm illustrée dans l'exemple de la Figure 3 est une courbe descendante. Dans une variante la courbe Cm peut être une courbe ascendante.

De même que pour l'analyse de la variation sur la deuxième échelle de temps décrite en référence à la Figure 2, les points considérés comme anormaux ont été exclus avant l'opération de moyennage. Les points considérés normaux correspondent à une moyenne hebdomadaire.

Tel qu'illustré à la figure 3, l'intervalle de temps prédéterminé ΔI1 peut comprendre les sept derniers points PI1 à PI7 considérés normaux, le premier point PI1 correspondant au point d'analyse Pa, et le point PI7 correspondant au point le plus ancien par rapport au point d'analyse Pa.

Tel qu'illustré à la Figure 3, afin de ne pas comparer le point d'analyse Pa à des évènements jugés trop anciens, le premier intervalle de temps ΔI1 est déterminé entre le point d'analyse Pa et le point PI1 le plus récent de la ligne de base LI long terme. Ce premier intervalle de temps ΔI1 peut correspondre à une durée de sept jours. Dans le cas inverse, l'analyse de variations peut être suspendue.

Ainsi, en limitant l'intervalle de temps prédéterminé ΔI1 aux sept derniers points les plus récents par rapport au point d'analyse Pa, il ne peut y avoir plus de sept points entre le point d'analyse Pa et le dernier point PI7, ce qui permet de ne pas comparer un maximum ou minimum hebdomadaire par rapport à des évènements considérés trop anciens.

Les points PI1 à PI7 de l'intervalle de temps prédéterminé ΔI1 sont comparés à une ligne de base LI dite long terme.

Dans l'exemple illustré à la Figure 3, la ligne de base LI dite long terme correspond aux vingt-huit derniers points considérés normaux de la courbe Cm précédant le point PI7. La ligne de base LI dite long terme dans l'exemple de la Figure 3 est ainsi comprise entre le point PI7 et un point PI35.

Dans une variante de la présente invention, la ligne de base LI dite long terme pourrait comprendre plus ou moins de vingt-huit points, du moins plus de points que la ligne de base Lm dite moyen terme.

Dans une variante, la ligne de base LI dite long terme ne peut comprendre plus de cinquante-six points afin d'éviter que des événements considérés comme trop anciens soient pris en considération.

L'analyse de variation sur la troisième échelle de temps LI dite « à long terme » se fait entre le maximum, minimum ou moyenne de points considérés normaux sur un intervalle de temps prédéterminé ΔI1 et la ligne de base long terme LI ou le maximum, minimum ou moyenne de points considérés normaux sur un intervalle de temps prédéterminé ΔI3. Les points de la courbe Cm compris dans l'intervalle de temps prédéterminé ΔI1 sont comparés à des points de la courbe Cm compris dans un intervalle de temps prédéterminé ΔI2 ou ΔI3.

L'intervalle de temps prédéterminé ΔI2 comprend les points entre PI7 et PIn, PIn correspondant au point le plus ancien par rapport à Pa. A titre d'illustration, ΔI2 sur la Figure 3 représente un intervalle comprenant cinquante-six points entre PI7 et PIn.

L'intervalle de temps prédéterminé ΔI3 comprend les sept derniers points considérés normaux de la courbe Cm à partir du point PI35 qui est le point le plus ancien de la ligne de base long terme. Dans l'exemple de la Figure 3, l'intervalle de temps prédéterminé ΔI3 comprend ainsi les points PI28 à PI35.

Lorsque la courbe Cm est descendante comme dans l'exemple de la Figure 3, le minimum des points PI1 à PI7 de l'intervalle de temps prédéterminé ΔI1 peut être comparée au maximum des points PI28 à PI35 de l'intervalle de temps prédéterminé ΔI3.

Dans une variante, la moyenne des points PI1 à PI7 de l'intervalle de temps prédéterminé ΔI1 peut être comparée à la moyenne des points PI28 à PI35 de l'intervalle de temps prédéterminé ΔI3.

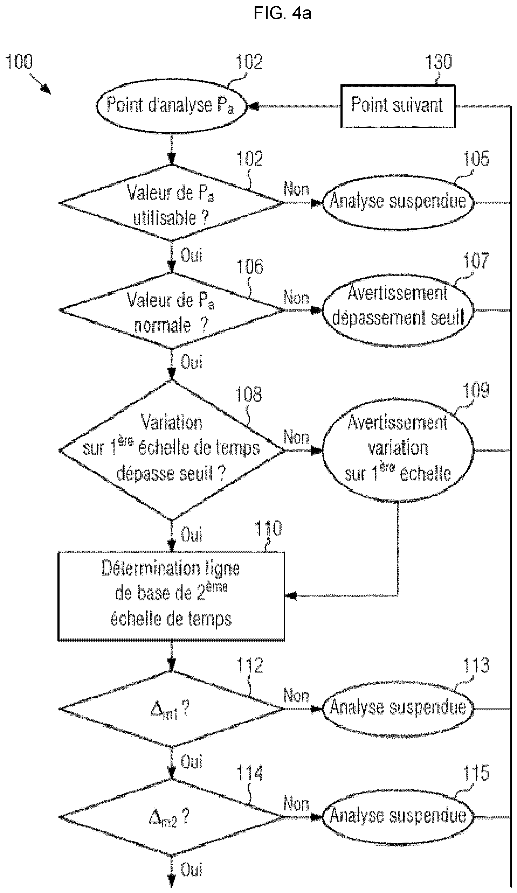

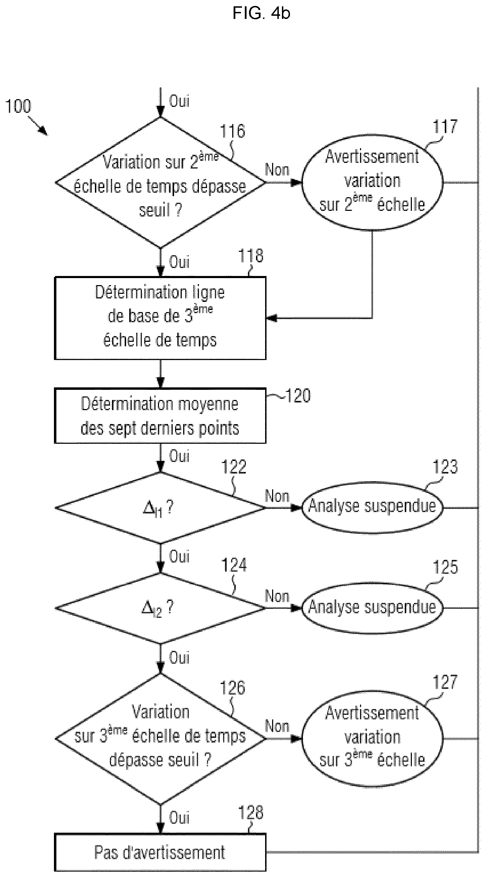

Les Figures 4a et 4b illustrent un organigramme 100 représentatif de l'analyse des variations et des valeurs seuils sur trois échelles de temps différentes selon la présente invention. L'organigramme 100 est représenté en deux figures 4a, 4b uniquement par soucis de clarté des dessins. La figure 4b illustre la suite des étapes représentées à la figure 4a. Ainsi, l'étape 114 de la figue 4a est suivie par l'étape 116 illustrée à la figure 4b.

L'organigramme 100 comprend des étapes mise en œuvre par l'unité de traitement 2 du dispositif de surveillance 4 de fonctionnement d'une sonde d'un dispositif cardiaque actif implantable 1 tel que décrit ci-dessus. Il s'agit ainsi de l'analyse de variations de valeurs d'un paramètre caractérisant la sonde implantable 7. De ce fait, les éléments avec les mêmes références numériques déjà utilisées pour la description des Figures 1 à 3 ne seront pas décrits à nouveau en détail, et référence est faite à leurs descriptions ci-dessus.

A une première étape 102 de l'analyse des variations, le point d'analyse Pa est prise en compte par l'unité de traitement 2.

A une étape 104, il est déterminé si la valeur du point d'analyse Pa correspond à une valeur utilisable. Si la valeur du point d'analyse Pa dépasse une valeur limite prédéfinie, elle est qualifiée de « point non utilisable » c'est-à-dire à un point dont la valeur est en dehors d'une gamme de valeurs viables ou dont la valeur n'est pas disponible. Dans ce cas, l'analyse est suspendue à une étape 105. Un point suivant sera alors considéré pour le point d'analyse Pa à une étape 130.

Si la valeur du point d'analyse Pa est considérée comme utilisable, l'analyse se poursuit.

A une étape 106, il est déterminé si la valeur du point d'analyse Pa correspond à une valeur qui peut être considérée de « valeur normale ». A cette fin, la valeur du point d'analyse est comparée à un seuil limite, maximal ou minimal prédéfini. Si la valeur du point d'analyse Pa dépasse le seuil maximal ou minimal prédéfini, la valeur du point d'analyse Pa est considérée comme anormale. Dans ce cas, un avertissement indiquant un dépassement de seuil est levé à une étape 107 et un point suivant sera alors considéré pour le point d'analyse Pa.

Si la valeur du point d'analyse Pa est considérée comme normale, l'analyse se poursuit à une étape 130.

A une étape 108, il est déterminé si la variation sur la première échelle de temps dépasse un seuil limite prédéterminé, en l'occurrence une variation limite. La première échelle de temps peut se rapporter à une journée. La variation sur la première échelle de temps correspond alors à la variation entre un point maximal et un point minimal d'une même journée.

Si le seuil prédéterminé pour la première échelle de temps est effectivement dépassé, un avertissement indiquant un dépassement relatif à une variation est levé à une étape 109.

Que le seuil prédéterminé pour la première échelle de temps ait été dépassé ou non à l'étape 108, il est déterminé à une étape 110 une ligne de base sur une deuxième échelle de temps, qui est différente de la première échelle de temps. La deuxième échelle de temps peut se rapporter à une durée d'une semaine.

A une étape 112, il est déterminé si le nombre de jours entre le point d'analyse Pa et le point Pm1 le plus récent de la ligne de base Lm de la deuxième échelle dite « moyen terme » (voir Figure 2) est compris dans l'intervalle de temps prédéfini Δm1. De préférence, Δm1 est égal à sept jours.

Si ce n'est pas le cas, l'analyse est suspendue à une étape 113. Un point suivant sera alors considéré pour le point d'analyse Pa à une étape 130.

Autrement, l'analyse se poursuit.

A une étape 114, il est déterminé si le nombre de jours entre le point Pm1 le plus récent et le point Pm le plus ancien de la ligne de base Lm de la deuxième échelle dite « moyen terme » (voir Figure 2) est compris dans l'intervalle de temps prédéfini Δm2. De préférence, Δm2 est égal à quatorze jours.

Si ce n'est pas le cas, l'analyse est suspendue à une étape 115. Un point suivant sera alors considéré pour le point d'analyse Pa à une étape 130.

Autrement, l'analyse se poursuit.

A une étape 116, il est déterminé si la variation sur la deuxième échelle de temps dépasse un seuil limite prédéterminé, en l'occurrence une variation limite.

Si le seuil prédéterminé pour la deuxième échelle de temps est effectivement dépassé, un avertissement indiquant un dépassement relatif à une variation est levé à une étape 117.

Que le seuil prédéterminé pour la deuxième échelle de temps ait été dépassé ou non à l'étape 116, il est déterminé à une étape 118 une ligne de base sur une troisième échelle de temps, qui est différente de la première échelle de temps et de la deuxième échelle de temps. La troisième échelle de temps peut se rapporter à un mois et est considéré comme « long terme ».

A une étape 120, une moyenne des sept derniers points de la ligne de base LI dite long terme (c'est-à-dire une moyenne des sept points à partir du point le plus ancien de la ligne de base LI dite long terme - voir Figure 3) est déterminée. Dans une variante, un point maximal ou un point minimal des sept derniers points de la ligne de base LI dite long terme peut être déterminé à l'étape 120.

A une étape 122, il est déterminé si le nombre de jours entre le point d'analyse Pa correspondant au point PI1 le plus récent et le point PIn le plus ancien considéré pour l'analyse sur la troisième échelle temps (voir Figure 3) est compris dans l'intervalle de temps prédéfini ΔI1. De préférence, ΔI1 est égal à sept jours.

Si ce n'est pas le cas, l'analyse est suspendue à une étape 123. Un point suivant sera alors considéré pour le point d'analyse Pa à une étape 130.

Autrement, l'analyse se poursuit.

A une étape 124, il est déterminé si le nombre de jours entre le point PI1 le plus récent et le point PI le plus ancien de la ligne de base LI de la troisième échelle dite « long terme » (voir

Figure 3) est compris dans l'intervalle de temps prédéfini ΔI2. De préférence, ΔI2 est égal à cinquante-six jours.

Si ce n'est pas le cas, l'analyse est suspendue à une étape 125. Un point suivant sera alors considéré comme point d'analyse Pa à une étape 130.

Autrement, l'analyse se poursuit.

A une étape 126, il est déterminé si la variation sur la troisième échelle de temps dépasse un seuil limite prédéterminé, en l'occurrence une variation limite.

Si le seuil prédéterminé pour la troisième échelle de temps est effectivement dépassé, un avertissement indiquant un dépassement relatif à une variation est levé à une étape 127.

Sinon, aucun avertissement n'est levé à une étape 128.

Dans les deux cas, que le seuil prédéterminé ait été dépassé ou non, l'analyse se poursuit en prenant en considérant un point suivant pour le point d'analyse Pa à une étape 130.

L'analyse de variation illustrée par l'organigramme 100 comprend ainsi des analyses de variations successives sur différentes échelles de temps, de l'échelle de temps la plus courte vers l'échelle de temps la plus longue.

La Figure 5 représente un organigramme 300 relatif à un déclenchement d'alerte en fonction des avertissements levés aux étapes 107, 109, 117 et 127 de l'organigramme 100.

L'organigramme 300 comprend des étapes mise en œuvre par l'unité de traitement 2 du dispositif de surveillance 4 de fonctionnement d'une sonde d'un dispositif cardiaque actif implantable 1 tel que décrit ci-dessus. De ce fait, les éléments avec les mêmes références numériques déjà utilisées pour la description des Figures 1 à 4 ne seront pas décrits à nouveau en détail, et référence est faite à leurs descriptions ci-dessus.

L'organigramme 300 illustre comment sont combinés les différents avertissements précédemment levés au cours des étapes 107, 109, 117 et 127 de l'organigramme 100 afin d'optimiser la sensibilité et la spécificité des alertes envoyées au médecin. Autrement dit, afin de ne déclencher que des alertes justifiées.

Selon la présente invention, un avertissement est différent d'une alerte. L'alerte est d'emblée communiquée au médecin pour indiquer une potentielle défaillance de sonde, par un message visuel ou sonore par exemple. Un avertissement n'est pas nécessairement communiqué au médecin. Toutefois, tel qu'il sera expliqué dans ce qui suit, la concomitance d'avertissements peut mener au déclenchement d'une alerte.

Ainsi, alors qu'une analyse de valeurs supérieures ou inférieures à un seuil limite peut permettre de lever d'emblée une alerte (par exemple dans le cas d'une impédance ou une continuité), une analyse de variation de ce même paramètre devra, elle, passer par une étape d'avertissement.

Deux types d'avertissement peuvent être pris en compte par l'unité de traitement 2 du dispositif de surveillance 4 de la présente invention : les avertissements de variations (à une étape 301) et les avertissements de seuils (à une étape 302).

Tel que décrit en référence à l'étape 107 de la figure 4a, un avertissement de seuil correspond au franchissement d'un seuil limite par la valeur du point d'analyse Pa.

Tel que décrit en référence aux étapes 109 de la figure 4a et aux étapes 117 et 127 de la figure 4b, un avertissement de variations, correspond au franchissement d'un seuil limite par la variation selon une échelle de temps de la valeur d'un paramètre caractérisant la sonde implantable 7.

Au moins un seuil limite est déterminé pour chaque paramètre caractérisant la sonde implantable 7.

Plusieurs seuils limites peuvent être déterminés pour un même paramètre. Ainsi, un paramètre peut avoir un premier seuil limite dont le dépassement engendre un avertissement, et un deuxième seuil limite dont le dépassement engendre une alerte.

Selon la présente invention, les seuils limites des paramètres caractérisant la sonde peuvent être classés en deux groupes.

Le premier groupe regroupe les seuils limites pour lesquels l'unité d'alerte du dispositif de surveillance 4 est configurée pour émettre une alerte en cas de dépassement d'un seuil limite d'un seul paramètre. Par exemple, les seuils limites relatifs à l'impédance de sonde, à la continuité de la sonde et au nombre d'extrasystoles totales font partie du premier groupe.

Le second groupe regroupes les seuils limites pour lesquels l'unité d'alerte du dispositif de surveillance 4 est configurée pour émettre une alerte en cas de dépassement concomitant des seuils limites d'au moins deux différents paramètres. Par exemple, les seuils limites relatifs à l'amplitude d'un signal de détection, au pourcentage de détection, au seuil de stimulation, au nombre d'extrasystoles isolées, au nombre de fibrillations ventriculaires traitées, au nombre de fibrillations ventriculaires soutenues mais non traitées, et au nombre de fibrillations ventriculaires non soutenues font partie du deuxième groupe.

Notons qu'un seuil limite d'un paramètre assigné au deuxième groupe peut être transféré dans le premier groupe si le dépassement dudit seuil limite a lieu successivement un nombre prédéterminé de fois.

Notons également que le seuil limite lui-même pour lever une alerte peut varier. C'est le cas par exemple pour l'impédance d'une sonde du ventricule gauche: un avertissement peut être levé pour un vecteur unipolaire sur un seuil limite plus bas que pour un vecteur bipolaire.

Tel qu'illustré à la Figure 5, si à une étape 302 de l'organigramme 300, il est détecté qu'un avertissement de seuil a été levé (à l'étape 107 de l'organigramme 100), il est déterminé à une étape 304 si l'avertissement de seuil levé se rapporte à une valeur d'un paramètre classé dans le premier groupe.

Si tel est le cas, cette condition suffit à ce qu'une alerte soit émise par l'unité d'alerte du dispositif de surveillance 4 à une étape 306. Comme expliqué précédemment en référence aux seuils limites du premier groupe, une analyse de valeurs dépassant un seuil limite peut en effet permettre de lever d'emblée une alerte (par exemple dans le cas d'une impédance ou une continuité). Ainsi, une valeur très haute ou très basse d'un paramètre (par exemple une impédance de sonde supérieure à 2000 ohms) est en tant que telle caractéristique d'un problème de sonde (en faveur d'une fracture). Ce facteur peut donc se suffire à lui-même pour déclencher une alerte indiquant un potentiel défaut de sonde.

Sinon, il est vérifié à une étape 308 si l'avertissement de seuil levé se rapporte à un paramètre comprenant un deuxième seuil limite dont le dépassement est susceptible de déclencher une alerte. En effet, tel qu'expliqué ci-dessus, certains paramètres, pour les extrasystoles totales par exemple, peuvent avoir un premier seuil limite dont le dépassement engendre un avertissement, et un deuxième seuil limite dont le dépassement engendre une alerte. Dans ce cas, il est vérifié à une étape 310 si la valeur du point d'analyse franchit le deuxième seuil limite. Dans l'affirmative, une alerte est déclenchée à l'étape 306.

Les cas non couverts ci-dessus par les avertissements pouvant à eux-seuls générer une alerte sont décrits dans ce qui suit.

Tel qu'illustré par l'organigramme 300 à l'étape 301, un avertissement de variations d'un paramètre ne se suffit pas à lui-même pour lever une alerte.

Ce paramètre a donc besoin d'avoir au moins un second paramètre concomitant pour qu'une alerte puisse être déclenchée.

Il est ainsi déterminé à une étape 312 si un avertissement relatif à un second paramètre a été détecté de manière concomitante à l'avertissement de l'étape 301. Ce second paramètre peut ne pas être non plus suffisant dans le cas où il reflète le même problème (par exemple la proportion de détection et l'amplitude de détection). On dit alors que le premier et le second paramètre sont « liés ».

C'est pourquoi à une étape 314 il est déterminé si le premier paramètre et le deuxième paramètre sont liés entre eux.

S'ils ne sont pas liés entre eux, alors la concomitance d'un avertissement relatif à un premier paramètre avec un avertissement relatif à un second paramètre, non-lié au premier paramètre, engendre le déclenchement d'une alerte à l'étape 306.

Il est donc nécessaire d'avoir au moins un second paramètre non-lié, comme par exemple le nombre d'épisodes de fibrillation ventriculaire par jour ou le seuil de stimulation, pour lever une alerte.

Les couples de paramètres caractérisant la sonde qui ne se suffisent pas entre eux pour déclencher une alerte car ils sont « liés » sont illustrés au moyen des cases grisées de la Figure 6, qui sera davantage décrite ci-après.

Trois couples de paramètres dits « liés » sont ainsi définis. Le premier couple correspond à la détection/jour et à l'amplitude de l'onde. Le deuxième couple correspond à un épisode soutenu et à un épisode non traité. Enfin, le troisième couple correspond à des extrasystoles isolées et à des extrasystoles totales.

S'il est déterminé à l'étape 314 de l'organigramme 300 que les deux paramètres sont liés, ou même qu'à l'étape 312 un avertissement relatif à un deuxième paramètre n'avait pas été détecté de manière concomitante, il est déterminé à une étape 316 si l'avertissement relatif au premier paramètre (celui de l'étape 301) est déclenché chaque jour.

A cette fin, les avertissements relatifs au premier paramètre sont sauvegardés dans une unité de mémoire d'unité de traitement 2.

Il faut noter que l'analyse des différents paramètres se fait simultanément. Lorsque l'un des paramètres lève un avertissement, celui-ci reste actif pendant une période prédéterminée, par exemple de sept jours.

Si l'avertissement est levé plusieurs jours de suite, alors il restera actif pendant les sept jours suivant la fin de sa levée.

Si plusieurs avertissements de différents paramètres sont actifs au même moment (c'est-à-dire de manière concomitante), cela peut activer une alerte.

Ce système de concomitance des alertes permet de détecter une défaillance pouvant se déclarer de différentes façons, à différents moments.

A une étape 318, il est déterminé si l'avertissement a été déclenché il a y plus de sept jours. Si tel est le cas, ledit avertissement est désactivé (éteint) à l'étape 320. Sinon, l'analyse continue à l'étape 322 en prenant en considération le point suivant.

La Figure 6 représente un tableau de pondération de la suffisance des avertissements entre eux.

Afin de calculer la suffisance des avertissements entre eux, notamment à l'étape 314 de l'organigramme 300, un système de pondération par binôme est mis en place.

Tous les avertissements relatifs aux paramètres caractérisant la sonde apparant dans une journée sont classés par ordre alphabétique.

Les cases rayées de la Figure 6 représentent un seul avertissement, et non pas deux avertissements levés d'un même paramètre, comme la variation sur deux échelles différents d'un même paramètre.

Les « poids » attribués à chaque binôme dans le tableau sont additionnés. Si le résultat de ladite addition est supérieur et différent de 3, l'alerte se lève à l'étape 306 de l'organigramme 300 (voir Figure 5).

Il faut alors se placer sur la ligne du premier paramètre, puis additionner le poids de chacun des binômes formés au propre poids du premier paramètre (indiqué dans les cases hachurées). Des exemples sont fournis ci-après.

Dans un premier exemple, le premier paramètre correspond à l'impédance et le deuxième paramètre correspond au seuil de stimulation. Pour le premier exemple, il faut alors se placer sur la ligne de l'impédance, et additionner le propre poids de l'impédance (c'est-à-dire 2, voir la case hachurée) et le poids du binôme formé avec le seuil de stimulation (c'est-à-dire 4). Le résultat de l'addition, qui est 6, est supérieure à 3 : une alerte est donc levée.

Dans un deuxième exemple, le premier paramètre correspond à l'amplitude du signal et le deuxième paramètre correspond au proportion journalière de signal détecté, c'est-à-dire la proportion journalière de signal en rythme spontané. Pour le deuxième exemple, il faut alors se placer sur la ligne de l'amplitude de l'onde, et additionner le propre poids de l'amplitude de l'onde (c'est-à-dire 2, voir la case hachurée) et le poids du binôme formé avec la détection/jour (c'est-à-dire 1). Le résultat de l'addition étant égale à 3, l'alerte n'est pas levée.

Dans un mode de réalisation de l'invention, le dispositif de surveillance de fonctionnement d'une sonde prend en considération au moins deux paramètres différents caractérisant la sonde.

Dans un autre mode de réalisation de l'invention, le dispositif de surveillance de fonctionnement d'une sonde prend en considération au moins trois paramètres différents caractérisant la sonde. Ainsi, il est décrit ci-après un troisième exemple dans lequel trois paramètres différents sont pris en compte.

Dans le troisième exemple, le premier paramètre correspond à l'amplitude de l'onde, le deuxième paramètre correspond à la détection/jour et le troisième paramètre correspond à la continuité. Pour le troisième exemple, il faut alors se placer sur la ligne de l'amplitude de l'onde, et additionner le propre poids de l'amplitude de l'onde (c'est-à-dire 2, voir la case hachurée), le poids du binôme formé avec la détection/jour (c'est-à-dire 1) et le poids du binôme formé avec la continuité (c'est-à-dire 4). Le résultat de l'addition étant égale à 7, c'est-à-dire supérieur à 3, l'alerte est levée.

La présente invention permet ainsi la prise en compte de paramètres multiples (électriques et rythmiques) caractéristiques d'une sonde implantable sur différentes échelles de temps afin d'améliorer la prédiction d'une défaillance de la sonde implantable.

## Revendications

1. Dispositif de surveillance (4) de fonctionnement d'une sonde (7) d'un dispositif cardiaque actif implantable (1), en particulier un défibrillateur automatique implantable ou un défibrillateur pour resynchronisation cardiaque, comprenant :
un dispositif de détermination de paramètres pour déterminer des valeurs d'une pluralité de paramètres caractérisant la sonde (7),
une unité de traitement (2) configurée pour déterminer des valeurs représentatives d'au moins un paramètre de la pluralité de paramètres caractérisant la sonde (7) en se basant sur au moins deux échelles de temps différentes, l'unité de traitement (2) étant en outre configurée pour comparer une valeur dite valeur d'analyse d'au moins un paramètre de la pluralité de paramètres caractérisant la sonde (7) avec les valeurs représentatives dudit paramètre.

2. Le dispositif de surveillance de fonctionnement d'une sonde d'un dispositif cardiaque actif implantable selon la revendication 1, dans lequel une première valeur représentative est une moyenne d'un premier nombre prédéfini de valeurs représentatives déterminées avant la valeur d'analyse qui est comparée au moyen de l'unité de traitement.

3. Le dispositif de surveillance de fonctionnement d'une sonde d'un dispositif cardiaque actif implantable selon la revendication 2, dans lequel une deuxième valeur représentative est une moyenne d'un deuxième nombre prédéfini de valeurs représentatives déterminées avant la valeur d'analyse qui est comparée au moyen de l'unité de traitement, ledit deuxième nombre prédéfini étant supérieur au premier nombre prédéfini.

4. Le dispositif de surveillance de fonctionnement d'une sonde d'un dispositif cardiaque actif implantable selon au moins une des revendications 1 à 3, dans lequel une troisième valeur représentative est une moyenne glissante basée sur une moyenne d'un troisième nombre prédéterminé de valeurs représentatives déterminées avant la valeur d'analyse comparée au moyen de l'unité de traitement, ladite moyenne d'un troisième nombre prédéterminé de valeurs correspondant à un paramètre de la pluralité des paramètres.

5. Le dispositif de surveillance de fonctionnement d'une sonde d'un dispositif cardiaque actif implantable selon au moins une des revendications 1 à 4, dans lequel l'unité de traitement est configurée lors de la détermination des valeurs représentatives de manière qu'une valeur parmi les valeurs de la pluralité de paramètres caractérisant la sonde qui dépasse une valeur limite prédéfinie n'est pas prise en compte.

6. Le dispositif de surveillance de fonctionnement d'une sonde d'un dispositif cardiaque actif implantable selon au moins une des revendications 1 à 5, dans lequel l'unité de traitement est configurée pour comparer la valeur d'analyse d'au moins un paramètre de la pluralité de paramètres caractérisant la sonde avec les valeurs représentatives dudit au moins un paramètre, la valeur la plus récente par rapport à la valeur d'analyse qui est prise en compte pour la détermination des valeurs représentatives étant comprise dans un premier intervalle de temps prédéterminé.

7. Le dispositif de surveillance de fonctionnement d'une sonde d'un dispositif cardiaque actif implantable selon au moins une des revendications 1 à 6, dans lequel l'unité de traitement est configurée pour comparer la valeur d'analyse d'au moins un paramètre de la pluralité de paramètres caractérisant la sonde avec les valeurs représentatives dudit au moins un paramètre, la valeur la plus ancienne par rapport à la valeur d'analyse qui est prise en compte pour la détermination des valeurs représentatives étant comprise dans un deuxième intervalle de temps prédéterminé.

8. Le dispositif de surveillance de fonctionnement d'une sonde d'un dispositif cardiaque actif implantable selon au moins une des revendications 1 à 7, dans lequel un paramètre est un paramètre parmi une amplitude du signal de détection, une continuité de la sonde, un pourcentage journalier de détection, un nombre de fibrillations ventriculaires non soutenues, un nombre de fibrillations ventriculaires non traitées, un nombre de fibrillations ventriculaires traitées, un nombre d'extrasystoles isolées, un nombre d'extrasystoles totales, une impédance de la sonde et un seuil de stimulation, ou/et
dans lequel la pluralité de paramètres caractérisant la sonde comprend au moins deux paramètres différents, en particulier au moins trois paramètres différents.

9. Le dispositif de surveillance de fonctionnement d'une sonde d'un dispositif cardiaque actif implantable selon au moins une des revendications 1 à 8, comprenant en outre une unité d'alerte pour émettre une alerte quand la valeur d'analyse dépasse de manière croissante ou décroissante une valeur limite d'au moins une valeur représentative ou/et un seuil limite d'au moins un paramètre de la pluralité des paramètres.

10. Le dispositif de surveillance de fonctionnement d'une sonde d'un dispositif cardiaque actif implantable selon la revendication 9, dans lequel chacun des paramètres de la pluralité de paramètres a respectivement un seuil limite, les seuils limites étant groupés dans: un premier groupe de seuils limites pour lequel l'unité d'alerte est configurée pour émettre une alerte en cas de dépassement d'un seuil limite d'un seul paramètre, ou un second groupe de seuils limites pour lequel l'unité d'alerte est configurée pour émettre une alerte en cas de dépassement concomitant des seuils limites d'au moins deux différents paramètres.

11. Le dispositif de surveillance de fonctionnement d'une sonde d'un dispositif cardiaque actif implantable selon la revendication 10, dans lequel un seuil limite d'un paramètre assigné au deuxième groupe est transféré dans le premier groupe si le dépassement dudit seuil limite a lieu successivement un nombre prédéterminé de fois, ou/et
dans lequel : les seuils limites relatifs à l'impédance de sonde, à la continuité de la sonde et au nombre d'extrasystoles totales font partie du premier groupe, et les seuils limites relatifs à l'amplitude d'un signal de détection, au pourcentage de détection, au seuil de stimulation, au nombre d'extrasystoles isolées, au nombre de fibrillations ventriculaires traitées, au nombre de fibrillations ventriculaires soutenues mais non traitées, et au nombre de fibrillations ventriculaires non soutenues font partie du deuxième groupe.

12. Le dispositif de surveillance de fonctionnement d'une sonde d'un dispositif cardiaque actif implantable selon la revendication 10 ou 11, dans lequel une valeur de pondération est attribuée à chaque paramètre du second groupe et dans lequel l'unité d'alerte est configurée pour déclencher une alerte quand la somme des valeurs de pondération des au moins deux paramètres dépasse un nombre prédéterminé.

13. Le dispositif de surveillance de fonctionnement d'une sonde d'un dispositif cardiaque actif implantable selon au moins une des revendications 10 à 12, dans lequel l'unité d'alerte comprend une unité de mémoire configurée pour sauvegarder un dépassement de seuil limite pendant une durée déterminée et pour le supprimer après l'écoulement de ladite durée déterminée.

14. Le dispositif de surveillance de fonctionnement d'une sonde d'un dispositif cardiaque actif implantable selon au moins une des revendications 10 à 13, dans lequel un paramètre de la pluralité de paramètres comprend un premier seuil limite et un deuxième seuil limite, le premier seuil limite faisant partie du premier groupe et le deuxième seuil limite faisant partie du deuxième groupe.

15. Le dispositif de surveillance de fonctionnement d'une sonde d'un dispositif cardiaque actif implantable selon au moins une des revendications 10 à 14, dans lequel des seuils limites parmi les seuils limites du deuxième groupe sont liés entre eux et d'autres ne sont pas liés entre eux, de manière que l'unité d'alerte est configurée pour déclencher une alerte en présence d'au moins deux dépassements de seuils limites parmi des seuils du deuxième groupe qui ne sont pas liés entre eux.

## Patentansprüche

1. Überwachungsvorrichtung (4) für den Betrieb einer Sonde (7) eines aktiven implantierbaren Herzgeräts (1), insbesondere eines implantierbaren automatischen Defibrillators oder eines Defibrillators zur kardialen Resynchronisation, umfassend:
eine Einrichtung zur Parameterbestimmung zur Bestimmung von Werten einer Vielzahl von Parametern, die die Sonde (7) charakterisieren,
eine Verarbeitungseinheit (2), die konfiguriert ist, repräsentative Werte von mindestens einem Parameter der Vielzahl von Parametern, die die Sonde (7) charakterisieren, auf der Grundlage von mindestens zwei unterschiedlichen Zeitskalen zu bestimmen, wobei die Verarbeitungseinheit (2) ferner konfiguriert ist, einen sogenannten Analysewert von mindestens einem Parameter der Vielzahl von Parametern, die die Sonde (7) charakterisieren, mit den repräsentativen Werten des betreffenden Parameters zu vergleichen.

2. Die Überwachungsvorrichtung für den Betrieb einer Sonde eines aktiven implantierbaren Herzgeräts gemäß Anspruch 1, wobei ein erster repräsentativer Wert ein Mittelwert einer ersten vordefinierten Anzahl von repräsentativen Werten ist, die vor dem Analysewert bestimmt wurden, der mittels der Verarbeitungseinheit verglichen wird.

3. Die Überwachungsvorrichtung für den Betrieb einer Sonde eines aktiven implantierbaren Herzgeräts gemäß Anspruch 2, wobei ein zweiter repräsentativer Wert ein Mittelwert einer zweiten vordefinierten Anzahl von repräsentativen Werten ist, die vor dem Analysewert bestimmt wurden, der mittels der Verarbeitungseinheit verglichen wird, wobei die zweite vordefinierte Anzahl größer ist als die erste vordefinierte Anzahl.

4. Die Überwachungsvorrichtung für den Betrieb einer Sonde eines aktiven implantierbaren Herzgeräts gemäß einem der Ansprüche 1 bis 3, wobei ein dritter repräsentativer Wert ein gleitender Mittelwert ist, der auf einem Mittelwert einer dritten vordefinierten Anzahl von repräsentativen Werten basiert, die vor dem Analysewert bestimmt wurden, der mittels der Verarbeitungseinheit verglichen wird, wobei der Mittelwert der dritten vordefinierten Anzahl von Werten einem Parameter der Vielzahl von Parametern entspricht.

5. Die Überwachungsvorrichtung für den Betrieb einer Sonde eines aktiven implantierbaren Herzgeräts gemäß einem der Ansprüche 1 bis 4, wobei die Verarbeitungseinheit bei der Bestimmung der repräsentativen Werte so konfiguriert ist, dass ein Wert unter den Werten der Vielzahl von Parametern, der einen vordefinierten Grenzwert überschreitet, nicht berücksichtigt wird.

6. Die Überwachungsvorrichtung für den Betrieb einer Sonde eines aktiven implantierbaren Herzgeräts gemäß einem der Ansprüche 1 bis 5, wobei die Verarbeitungseinheit konfiguriert ist, den Analysewert von mindestens einem Parameter der Vielzahl von Parametern, die die Sonde charakterisieren, mit den repräsentativen Werten dieses mindestens einen Parameters zu vergleichen, wobei der jüngste Wert im Verhältnis zum Analysewert, der zur Bestimmung der repräsentativen Werte berücksichtigt wird, innerhalb eines ersten vordefinierten Zeitintervalls liegt.

7. Die Überwachungsvorrichtung für den Betrieb einer Sonde eines aktiven implantierbaren Herzgeräts gemäß einem der Ansprüche 1 bis 6, wobei die Verarbeitungseinheit konfiguriert ist, den Analysewert von mindestens einem Parameter der Vielzahl von Parametern, die die Sonde charakterisieren, mit den repräsentativen Werten dieses mindestens einen Parameters zu vergleichen, wobei der älteste Wert im Verhältnis zum Analysewert, der zur Bestimmung der repräsentativen Werte berücksichtigt wird, innerhalb eines zweiten vordefinierten Zeitintervalls liegt.

8. Die Überwachungsvorrichtung für den Betrieb einer Sonde eines aktiven implantierbaren Herzgeräts gemäß einem der Ansprüche 1 bis 7, wobei ein Parameter ein Parameter aus der Gruppe bestehend aus: Amplitude des Detektionssignals, Sondenkontinuität, täglicher Detektionsprozentsatz, Anzahl nicht anhaltender ventrikulärer Fibrillationen, Anzahl unbehandelter ventrikulärer Fibrillationen, Anzahl behandelter ventrikulärer Fibrillationen, Anzahl isolierter Extrasystolen, Gesamtanzahl von Extrasystolen, Sondenimpedanz und Stimulationsschwelle ist, und/oder
wobei die Vielzahl von Parametern, die die Sonde charakterisieren, mindestens zwei unterschiedliche Parameter umfasst, insbesondere mindestens drei unterschiedliche Parameter.

9. Die Überwachungsvorrichtung für den Betrieb einer Sonde eines aktiven implantierbaren Herzgeräts gemäß einem der Ansprüche 1 bis 8, ferner umfassend eine Alarmeinheit, die konfiguriert ist, einen Alarm auszulösen, wenn der Analysewert einen Grenzwert von mindestens einem repräsentativen Wert oder/und einen Schwellenwert von mindestens einem Parameter der Vielzahl von Parametern überschreitet, sei es ansteigend oder abfallend.

10. Die Überwachungsvorrichtung für den Betrieb einer Sonde eines aktiven implantierbaren Herzgeräts gemäß Anspruch 9, wobei jeder Parameter der Vielzahl von Parametern jeweils einen Schwellenwert aufweist, wobei die Schwellenwerte in folgende Gruppen unterteilt sind:
eine erste Gruppe von Schwellenwerten, bei der die Alarmeinheit konfiguriert ist, einen Alarm bei Überschreitung eines Schwellenwerts eines einzelnen Parameters auszulösen, und
eine zweite Gruppe von Schwellenwerten, bei der die Alarmeinheit konfiguriert ist, einen Alarm bei gleichzeitiger Überschreitung der Schwellenwerte von mindestens zwei unterschiedlichen Parametern auszulösen.

11. Die Überwachungsvorrichtung für den Betrieb einer Sonde eines aktiven implantierbaren Herzgeräts gemäß Anspruch 10, wobei ein Schwellenwert eines Parameters, der der zweiten Gruppe zugeordnet ist, in die erste Gruppe übertragen wird, wenn die Überschreitung dieses Schwellenwerts eine vordefinierte Anzahl aufeinanderfolgender Male auftritt, und/oder
wobei: die Schwellenwerte bezüglich der Sondenimpedanz, der Sondenkontinuität und der Gesamtanzahl von Extrasystolen zur ersten Gruppe gehören, und die Schwellenwerte bezüglich der Amplitude eines Detektionssignals, des Detektionsprozentsatzes, der Stimulationsschwelle, der Anzahl isolierter Extrasystolen, der Anzahl behandelter ventrikulärer Fibrillationen, der Anzahl anhaltender aber unbehandelter ventrikulärer Fibrillationen sowie der Anzahl nicht anhaltender ventrikulärer Fibrillationen zur zweiten Gruppe gehören.

12. Die Überwachungsvorrichtung für den Betrieb einer Sonde eines aktiven implantierbaren Herzgeräts gemäß Anspruch 10 oder 11, wobei jedem Parameter der zweiten Gruppe ein Gewichtungswert zugewiesen ist und wobei die Alarmeinheit konfiguriert ist, einen Alarm auszulösen, wenn die Summe der Gewichtungswerte von mindestens zwei Parametern einen vordefinierten Wert überschreitet.

13. Die Überwachungsvorrichtung für den Betrieb einer Sonde eines aktiven implantierbaren Herzgeräts gemäß einem der Ansprüche 10 bis 12, wobei die Alarmeinheit eine Speichereinheit umfasst, die konfiguriert ist, eine Schwellenwertüberschreitung für eine bestimmte Dauer zu speichern und diese nach Ablauf der bestimmten Dauer zu löschen.

14. Die Überwachungsvorrichtung für den Betrieb einer Sonde eines aktiven implantierbaren Herzgeräts gemäß einem der Ansprüche 10 bis 13, wobei ein Parameter der Vielzahl von Parametern einen ersten Schwellenwert und einen zweiten Schwellenwert umfasst, wobei der erste Schwellenwert zur ersten Gruppe gehört und der zweite Schwellenwert zur zweiten Gruppe gehört.

15. Die Überwachungsvorrichtung für den Betrieb einer Sonde eines aktiven implantierbaren Herzgeräts gemäß einem der Ansprüche 10 bis 14, wobei einige Schwellenwerte der zweiten Gruppe miteinander verknüpft sind und andere nicht, sodass die Alarmeinheit konfiguriert ist, einen Alarm auszulösen, wenn mindestens zwei Überschreitungen von Schwellenwerten aus der zweiten Gruppe auftreten, die nicht miteinander verknüpft sind.

## Claims

1. A device (4) for monitoring operation of a probe (7) of an implantable active cardiac device (1), in particular an implantable automatic defibrillator or a defibrillator for cardiac resynchronization, comprising:
a parameter determining device for determining values of a plurality of parameters characterizing the probe (7);
a processing unit (2) configured to determine representative values of at least one of the plurality of parameters characterizing the probe (7) based on at least two different time scales, wherein the processing unit (2) is further configured to compare a value as an analysis value of at least one of the plurality of parameters characterizing the probe (7) with the representative values of said parameter.

2. The device for monitoring operation of a probe of an implantable active cardiac device according to claim 1, wherein a first representative value is an average of a first predefined number of representative values determined prior to the analysis value that is compared by means of the processing unit.

3. The device for monitoring operation of a probe of an implantable active cardiac device according to claim 2, wherein a second representative value is an average of a second predefined number of representative values determined prior to the analysis value that is compared by means of the processing unit, said second predefined number being greater than the first predefined number.

4. The device for monitoring operation of a probe of an implantable active cardiac device according to at least one of claims 1 to 3, wherein a third representative value is a rolling average based on an average of a third predetermined number of representative values determined prior to the analysis value compared by means of the processing unit, said average of a third predetermined number of representative values corresponding to one of the plurality of parameters.

5. The device for monitoring operation of a probe of an implantable active cardiac device according to at least one of claims 1 to 4, wherein the processing unit is configured during the determination of the representative values in such a way that one of the values of the plurality of parameters characterizing the probe that exceeds a predefined limit value is not taken into account.

6. The device for monitoring operation of a probe of an implantable active cardiac device according to at least one of claims 1 to 5, wherein the processing unit is configured to compare the analysis value of at least one of the plurality of parameters characterizing the probe with the representative values of the at least one parameter, the most recent value relative to the analysis value that is taken into account for the determination of the representative values being included within a first predetermined time interval.

7. The device for monitoring operation of a probe of an implantable active cardiac device according to at least one of claims 1 to 6, wherein the processing unit is configured to compare the analysis value of at least one of the plurality of parameters characterizing the probe with the representative values of the at least one parameter, the oldest value relative to the analysis value that is taken into account for the determination of the representative values being included within a second predetermined time interval.

8. The device for monitoring operation of a probe of an implantable active cardiac device according to at least one of claims 1 to 7, wherein a parameter is one of an amplitude of the detection signal, a continuity of the probe, a daily detection percentage, a number of non-sustained ventricular fibrillations, a number of untreated ventricular fibrillations, a number of treated ventricular fibrillations, a number of isolated extrasystoles, a number of total extrasystoles, an impedance of the probe, and a pacing threshold, and/or wherein the plurality of parameters characterizing the probe comprises at least two different parameters, in particular at least three different parameters.

9. The device for monitoring operation of a probe of an implantable active cardiac device according to at least one of claims 1 to 8, further comprising an alert unit for issuing an alert when the analysis value exceeds, in an increasing or decreasing manner, a limit value of at least one representative value and/or a limit threshold of at least one of the plurality of parameters.

10. The device for monitoring operation of a probe of an implantable active cardiac device according to claim 9, wherein each parameter of the plurality of parameters respectively has a limit threshold, the limit thresholds being grouped into: a first group of limit thresholds for which the alert unit is configured to issue an alert in case of exceeding a limit threshold of a single parameter; or a second group of limit thresholds for which the alert unit is configured to issue an alert in case of simultaneously exceeding the limit thresholds of at least two different parameters.

11. The device for monitoring operation of a probe of an implantable active cardiac device according to claim 10, wherein a limit threshold of a parameter assigned to the second group is transferred to the first group if exceedance of said limit threshold occurs successively a predetermined number of times, and/or
wherein: the limit thresholds relating to the impedance of the probe, the continuity of the probe, and the number of total extrasystoles are part of the first group; and the limit thresholds relating to the amplitude of a detection signal, the detection percentage, the pacing threshold, the number of isolated extrasystoles, the number of treated ventricular fibrillations, the number of sustained but untreated ventricular fibrillations, and the number of non-sustained ventricular fibrillations are part of the second group.

12. The device for monitoring operation of a probe of an implantable active cardiac device according to claim 10 or 11, wherein a weighting value is assigned to each parameter of the second group, and wherein the alert unit is configured to trigger an alert when a sum of the weighting values of the at least two parameters exceeds a predetermined number.

13. The device for monitoring operation of a probe of an implantable active cardiac device according to at least one of claims 10 to 12, wherein the alert unit comprises a memory unit configured to store a limit threshold exceedance for a determined duration and to delete it after the expiration of said determined duration.

14. The device for monitoring operation of a probe of an implantable active cardiac device according to at least one of claims 10 to 13, wherein a parameter of the plurality of parameters comprises a first limit threshold and a second limit threshold, the first limit threshold being part of the first group and the second limit threshold being part of the second group.

15. The device for monitoring operation of a probe of an implantable active cardiac device according to at least one of claims 10 to 14, wherein limit thresholds among the limit thresholds of the second group are linked to each other and others are not linked to each other, in such a way that the alert unit is configured to trigger an alert in the presence of at least two exceedances of threshold limits among thresholds of the second group that are not linked to each other.
